# EUROPEAN PATENT SPECIFICATION

(11) **EP 2 257 233 B1**
(45) Date of publication and mention of the grant of the patent: **02.11.2011**
(21) Application number: 09725315.7
(22) Date of filing: 24.03.2009
(51) Int. Cl.: A61B 17/70, A61B 17/00

(54) **STABILIZATION RODS**
STABILISIERUNGSSTANGEN
TIGES DE STABILISATION

(30) Priority: 24.03.2008 US 53924
(43) Date of publication of application: 08.12.2010
(73) Proprietor: INGENIUM, S.A., Panama City (PA)
(72) Inventor: RANDOL, David, S., Odessa FL 44556 (US); WALSH, David A., Reading MA 01867 (US); KNOBLOCH, Carl, A., Oviedo FL 32765 (US)
(74) Representative: Mitchell, Matthew Benedict David
(86) International application number: PCT/US2009/038073
(87) International publication number: WO 2009/120680

(56) References cited:
- WO-A-2007/090015
- WO-A-2007/090021
- US-A1- 2007 055 244
- US-A1- 2007 100 341

## Description

### FIELD OF THE INVENTION

The present invention relates generally to prostheses for treating spinal pathologies, and more specifically to stabilization rods for use with spinal fixation assemblies having an anchor for holding a fixation device and a stabilization rod.

### BACKGROUND OF THE INVENTION

Various methods of spinal immobilization have been used in the treatment of spinal instability and displacement. The most common treatment for spinal stabilization is immobilization of the joint by surgical fusion, or arthrodesis. This has been known for almost a century. In many cases, however, pseudoarthrosis occurs, particularly in cases involving fusion across the lumbosacral articulation and when more than two vertebrae are fused together. Early in the century, post operative external immobilization, such as through the use of splints and casts, was the favored method of spinal fixation. As surgical techniques became more sophisticated, various new methods of internal and external fixation were developed.

Internal fixation refers to therapeutic methods of stabilization that are wholly internal to the patient and include commonly known devices such as bone plates, screws, rods and pins. External fixation, in contrast, involves at least some portion of the stabilization device being located external to the patients' body. As surgical technologies and procedures became more advanced and the likelihood of infection decreased, internal fixation eventually became the favored method of immobilization since it is less restrictive on the patient.

Internal fixation of the spine may be used to treat a variety of disorders including kyphosis, spondylolisthesis and rotation, segmental instability, such as disc degeneration and/or fracture caused by disease, trauma, congenital defects and tumor diseases. One of the main challenges associated with internal spinal fixation is securing the fixation device to the spine without damaging the spinal cord. The pedicles of a vertebra are commonly used for fixation as they generally offer an area that is strong enough to hold the fixation device in place even when the patient suffers from degenerative instability such as osteoporosis.

Current fixation devices and hardware systems generally include a fixation device, such as a screw, a rod, and a body for fixing the position of the rod with respect to the screw, which in turn fixes the rod with respect to the spine. The present invention provides a novel rod and fixation device.

### BRIEF SUMMARY OF THE INVENTION

Disclosed is a rod for use with spinal fixation assemblies. The rod comprises a metal infrastructure comprising: a top portion, a bottom portion, and at least one lateral channel extending therethrough. The rod also comprises an elastomeric material at least partially encapsulating the metal infrastructure and substantially filling at least one lateral channel extending through the metal infrastructure.

Also disclosed is a rod for use with spinal fixation assemblies that comprises a metal infrastructure having a top portion and a bottom portion. The rod also comprises an elastomeric material partially circumferentially encapsulating the metal infrastructure such that the metal infrastructure and elastomeric material together form a rod at least a portion of which is generally cylindrical wherein part of the generally cylindrical portion of the rod has a surface formed by the elastomeric material and part of the generally cylindrical portion has a surface that is formed by the bottom portion of the metal infrastructure that is not encapsulated by the elastomeric material.

Also disclosed is a rod for use with spinal fixation assemblies that comprises a non-cylindrical metal infrastructure comprising a top surface, a bottom surface and an end. The rod also comprises an elastomeric material at least partially encapsulating the metal infrastructure to form a rod that has having a generally cylindrical portion. In addition, the center of gravity along at least a portion of the length of the metal infrastructure is not equidistant from the top surface and the bottom surface of the metal infrastructure.

Further disclosed is a spinal fixation assembly comprising a rod and a locking mechanism. The rod has a top portion and a bottom portion and comprises metal material and elastomeric material. The elastomeric material partially surrounds the metal material such that at least part of the surface of the bottom portion of the rod is formed by the metal material. The locking mechanism is configured to receive the rod and engage part of the surface of the bottom portion of the rod that is formed by the metal material.

The features of the present invention will be apparent with reference to the following description and attached drawings. In the description and drawings, particular embodiments of the invention have been disclosed in detail as being indicative of some of the ways in which the principles of the invention may be employed, but it is understood that the invention is not limited correspondingly in scope. Rather, the invention includes all changes, modifications and equivalents coming within the terms of the claims appended hereto.

Features that are described and/or illustrated with respect to one embodiment may be used in the same way or in a similar way in one or more other embodiments and/or in combination with or instead of the features of the other embodiments.

### BRIEF DESCRIPTION OF THE SEVERAL VIEWS OF THE DRAWINGS

Figures 1A-B are top perspective views of a rod and metal infrastructure;

Figures 1C-D are bottom perspective views of the rod and metal infrastructure of Figures 1A-B;

Figures 1E-F are side views of the rod and metal infrastructure of Figures 1A-B;

Figures 1G-H are views of a cross section of part of the rod and metal infrastructure of Figures 1A-B;

Figure 1I is a front side view of the rod of Figure 1A showing illustrating the curvature of the rod;

Figure 2 is a perspective view of the rod of Figures 1A-I and two locking mechanisms;

Figure 3 is a cross-sectional view of the rod of Figures 1A-I and a locking mechanism;

Figure 4 is a perspective view of an alternate metal infrastructure having an I-beam shaped cross section;

Figure 5 is a perspective view of an alternate metal infrastructure having a star shaped cross section;

Figure 6 is a perspective view of alternate metal infrastructure having a generally circular cross section;

Figure 7 is a view of an exemplary testing procedure for measuring the dynamic properties of a rod; and

Figure 8 is a chart showing finite element analysis testing results of various types of rods.

### DETAILED DESCRIPTION OF THE INVENTION

The invention relates to novel stabilization rods for use with locking mechanisms for spine stabilization. The stabilization rods preferably permit a metal to metal contact surface between the rod and locking mechanism while exhibiting bending properties that are different than a metal rod of the same size. This may be accomplished through a variety of designs, each of which includes a rod made of both metal and elastomeric material.

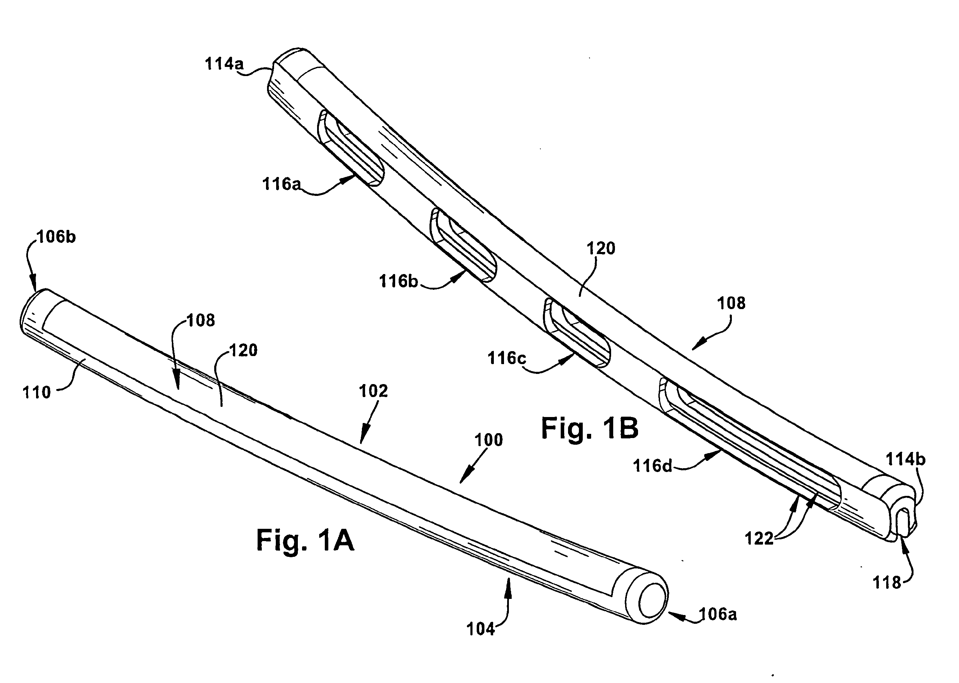

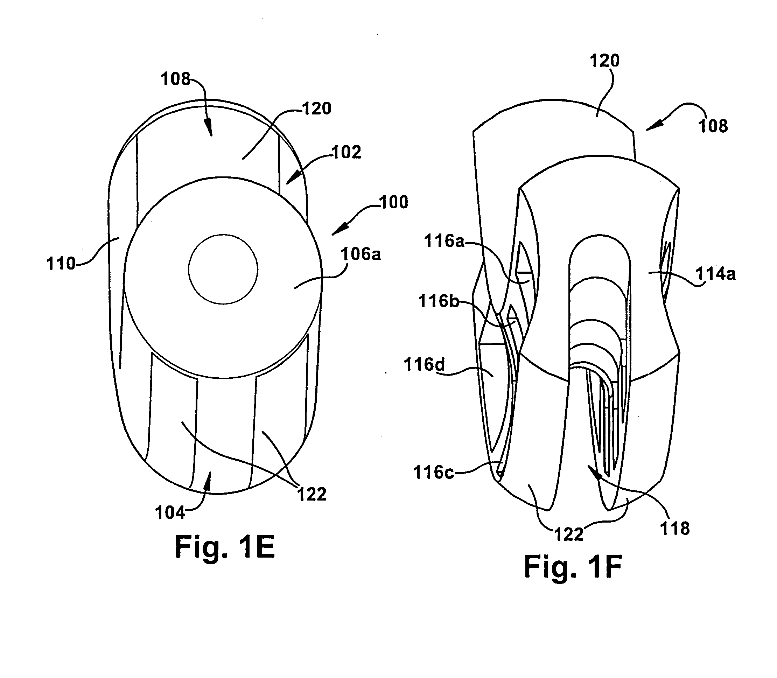

Turning initially to Figures 1A-F, perspective and side views of an exemplary stabilization rod are illustrated. The rod 100 has a top portion 102 and a bottom portion 104, as well as sides 106a and 106b. The rod 100 includes metal material 108 and elastomeric material 110. The metal material 108 may include, but is not limited to, titanium, titanium alloys (e.g., titanium/aluminum/vanadium (Ti/AlN) alloys), cobalt-chromium alloys, stainless steel, and combinations thereof, which may include mechanically compatible mixtures of the above materials, or other similar metal material(s). In the presently preferred embodiment, the metal material 108 is a Ti/Al/V alloy, such as Ti/6Al/4V ELI. The elastomeric material 110 is preferably one or more high strength polymers, such as Poly Ether Ether Keytone (PEEK), Poly Ether Ketone Ketone (PEKK), Poly Ether Ketone Ketone Ether Keyton (PEKKEK), Self-Reinforced Polyplenylene (SRP), Polyphenylsulfone (PPSU), Polysulfone (PSU), Ultra-High Molecular Weight Polyethylene (UHMWPE), or combinations thereof, or other similar material(s). In the presently preferred embodiment, the elastomeric material is PEKK.

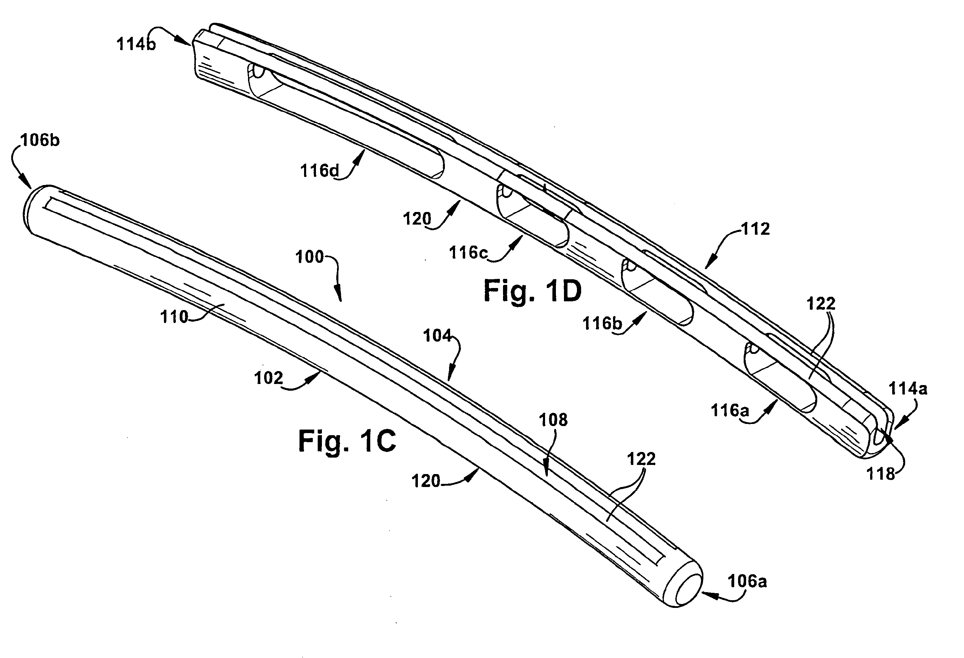

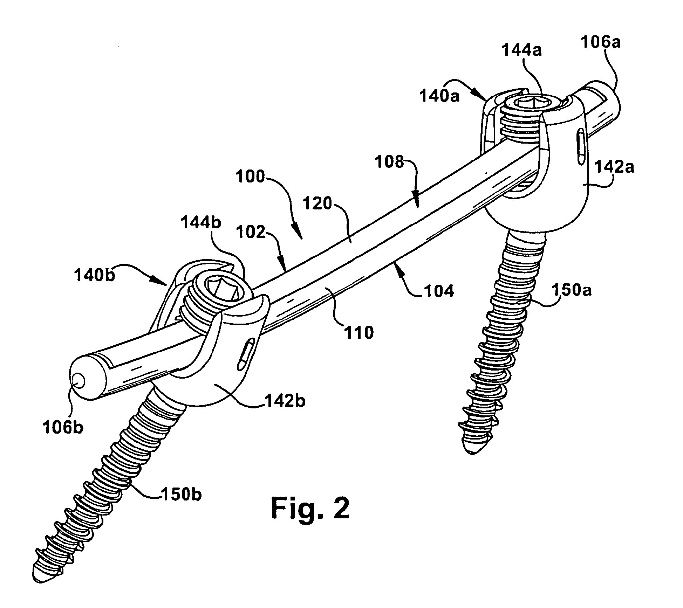

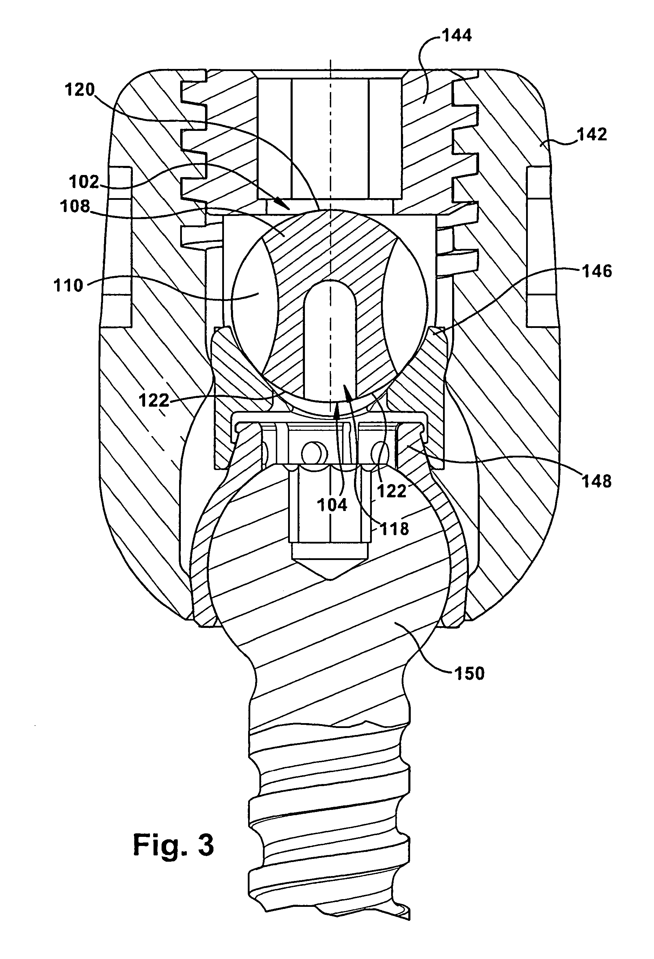

As shown in Figure 1A, part of the top portion 102 may comprise metal material 108, and other parts of the top portion 102 may comprise elastomeric material 110. Similarly, as shown in Figure 1C, part of the bottom portion 104 may comprise metal material 108, and other parts of the bottom portion 104 may comprise elastomeric material 110. Moreover, the rod 100 may be formed such that the elastomeric material 110 partially encapsulates the metal material 108, which may be accomplished by injection molding as will be understood by one of ordinary skill in the art. In addition, the top portion 102 and bottom portion 104 may each have zones of metal material 108 or elastomeric material 110. For example, Figure 1C illustrates multiple metal material 108 zones in the bottom portion 104. Moreover, it may be preferable to configure the rod 100 such that the metal material 108 portions are located along the rod 100 to facilitate metal on metal interaction with a locking mechanism, such as the locking mechanism 144 of Figures 2 and 3.

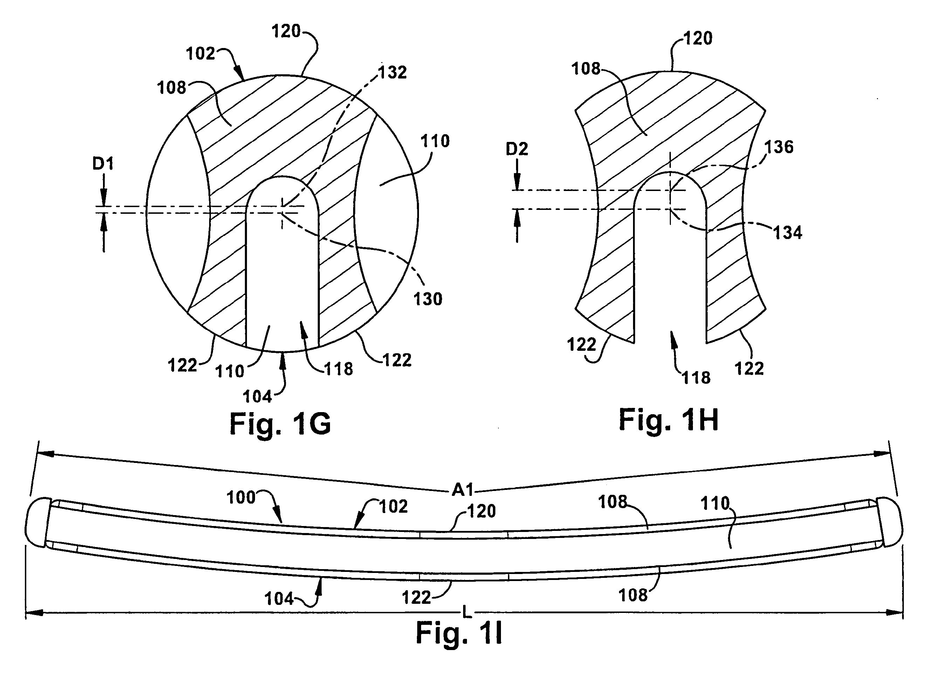

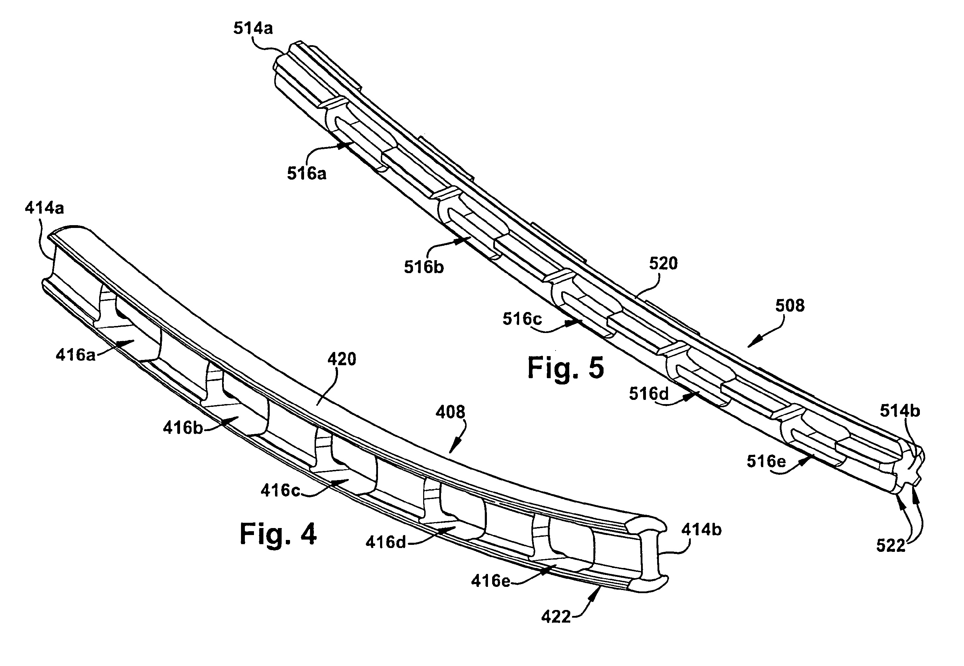

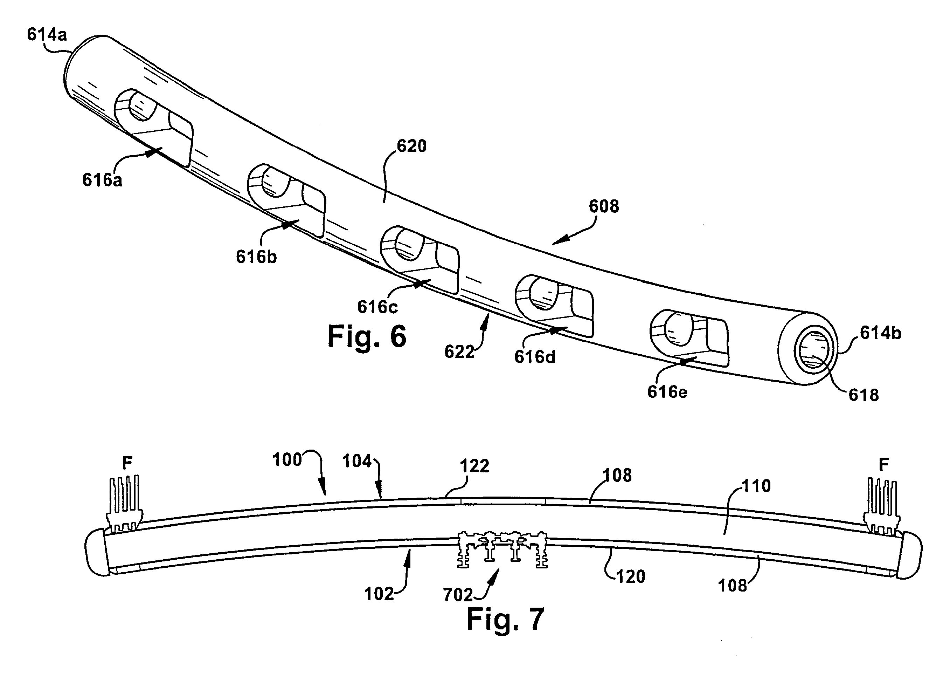

The metal material 108 may be in the form of a metal infrastructure 108, such as that illustrated in more detail in Figures 1B, 1D, 1F and 1H, or metal infrastructures 408, 508 or 608 as illustrated in Figures 4, 5 and 6, respectively. As used herein, the term "infrastructure" is not limited to an internal structure and is meant to also include the infrastructure 608 of Figure 6, even though the infrastructure 608 forms the exterior of the rod 600.

Turning first to Figures 1B, 1D, 1F and 1H, the metal infrastructure 108 includes a top portion 120 and a bottom portion 122 and has at least one lateral channel extending 116a-d therethrough. The metal infrastructure 108 is generally u-shaped. More specifically, a cross section of at least part of the metal infrastructure 108 resembles an upside down letter "U". The sides of the "U" may be flat or have curvature, such as the generally concave side surfaces of the metal infrastructure 108. In addition, the bottom portion 122 may have an axial channel 118 extending toward the center of the metal infrastructure and running along at least part of the length of the bottom portion 122. The axial channel 118 may intersect with one or more of the lateral channels 116a-d.

Thus, as shown in Figures 1A-1I, the rod 100 includes both the metal material 108 in the form of a U-shaped metal infrastructure 108 and elastomeric material 110. The elastomeric material 110 at least partially fills at least one of the lateral channels 116a-d of the metal infrastructure 108. In addition, the elastomeric material may substantially fill one or all of the lateral channels 116a-d.

At least one of the lateral channels 116a-d may be positioned such that it does not extend to the top portion 120 or bottom portion 122 of the metal infrastructure 108. In other words, the lateral channels 116a-d may entirely reside within the metal infrastructure 108. As shown, all of the lateral channels 116a-d are positioned such that they do not extend to the top portion 120 or bottom portion 122 of the metal infrastructure 108. In addition, the lateral channels 116a-d may have varying dimensions. For example, the lateral channel 116d may be larger than the lateral channel 116a. Varying the size and location of the lateral channels 116a-d may change the bending properties of the metal infrastructure 108, and thus, the rod 100.

Turning next to Figures 1G-H, a cross section of part of the rod 100 (Figure 1G) and a cross section of part of the metal infrastructure 108 (Figure 1H). In addition metal infrastructure 108 and elastomeric material 110, Figure 1G illustrates the geometric center 130 as well as the center of gravity 132 of the cross section. While the center of gravity 132 may be located at the geometric center 130, it is also possible for the center of gravity 132 and the geometric center 130 to not share the same location. For example, the geometric center 130 and the center of gravity 132 may separated by distance D1. In the example illustrated in Figure 1G, D1 is approximately 0.2 mm.

Similarly, Figure 1H illustrates the geometric center 134 and the center of gravity 136 of a cross section of the metal infrastructure 108. While the center of gravity 132 may be located at the geometric center 134, it is also possible for the center of gravity to be located such that it is not equidistant from the top portion 120 and the bottom portion 122 of the metal infrastructure 108. Specifically, D2 is the distance between the geometric center 134 and the center of gravity 136. D2 may be, for example, approximately 0.5 mm. In addition, the center of gravity 136 may be in the axial channel 118.

Turning next to Figure 1I, a side view of the rod 100 is illustrated. As will be understood by those skilled in the art, the rod 100 may be any size appropriate for the intended use of the rod 100. For example, when used for spinal stabilization, the rod 100 may have a diameter ranging from about 3 mm to about 7 mm. In addition, the rod may have a length L ranging from about 40 mm to about 180 mm. As can be seen, the top portion 102 of the rod 100 has a generally concave shape and the bottom portion 104 of the rod 100 has a generally convex shape due to the slight curvature of the rod 100. For example, the rod may be generally curved such that it forms an arc of approximately 10 degrees to approximately 30 degrees, as represented by angle A1. For the rod 100 illustrated in Figure 1I, angle A1 is 20 degrees. It will be understood by those of skill in the art, however, that the rod may be straight, and that the arc illustrated in Figure 1I may be any generally curved shape and is not limited to a circular arc.

Turning next to Figure 2, the rod 100 of Figures 1A-I is illustrated with two locking mechanisms 140a and 140b. The locking mechanisms 140a and 140b of Figure 2 may be and locking mechanism, such as any of the locking mechanisms described in U.S. Patent Application No.11/816,802. Moreover, for proper anatomical reference, "above" or "top" means posterior with respect to the patient and "below" or "bottom" means anterior with respect to the patient when the system of Figure 2 is used for spinal fixation. Thus, the bottom portion 104 of the rod 100 is anterior with respect to the patient and the top portion 102 of the rod 100 is posterior. Thus, the rod 100 is received by the locking mechanisms 140a and 140b as the rod 100 is moved in a posterior to anterior direction.

Each locking mechanism 140x includes a body 142x a fixation device 150x and a locking element 144. The rod 100 is received by the body 142x and locked into position with respect to the fixation device 150x. The locking element 144 prevents the rod 100 from moving upward in the locking mechanism 140x.

Turning next to Figure 3, a cross section of a rod 100 and an exemplary locking mechanism 140 is illustrated in greater detail. It will be understood by those skilled in the art that the rods of the present invention may be used with any of the variety of types of locking mechanisms known in the art. The exemplary locking mechanism 140 includes a body 142 that is configured to receive a fixation device 150, which may be, for example, a screw. The particular locking mechanism 140 illustrated also includes an insert 148 that engages the head of the fixation device 150. The insert 148 may be compressible to enable insertion into the body 140. Once inserted into the body 140, however, the insert 148 may expand to have a greater width.

When the fixation device 150 is inserted into body 140, the head of the fixation device 150 preferably engages the insert 148 in a snap-fit manner such that the insert 150 expands to accommodate the head. When the insert 148 and fixation device 150 combination is forced toward the bottom portion of the body 140, the body 140 engages the sides of the insert 148, causing the insert 148 to more tightly engage the head of the fixation device 150 and preventing the insert 148 and the fixation device 150 from exiting the body 142.

The body 142 also includes a side portion that is configured to receive the rod 100, such as by way of a channel that enables placement of the rod 100 by either sliding the rod 100 through the side portion of the body 142 or by inserting the rod 100 into the channel through the top portion of the body 142. The body 142 is also configured to receive a rod seat 146, for example, through a hole in the bottom or top of the body 142. The rod seat 146 is preferably inserted into the body 142 prior to insertion of the rod 100 such that the rod seat 146 is eventually positioned between the rod 100 and the insert 148.

The rod seat 146 may have a tapered portion for receiving the rod 100. The tapered portion of the rod seat 146 may be configured to engage rods of varying diameters. For example, the tapered portion of the rod seat 146 may have multiple curvatures on each side of the rod seat 146 that provide varying surfaces for contacting rods of varying diameters. As can be seen in Figure 3, the bottom portion 104 of the rod 100 contacts the rod seat 146. Preferably, the metal material, which may be in the form of a metal infrastructure 108, contacts the rod seat 146, which is also preferably metal. The metal-on-metal interface may be more stable than a metal-on-elastomeric material interface. As shown, the bottom portion 122 of the metal infrastructure 108 engages the taper of the rod seat 146. It will be understood by those of skill in the art that the contact surface between the rod 100 and the locking mechanism 140 may be achieved by other means and may be flat as opposed to tapered. Accordingly, it may be desirable in such circumstances to use a rod having a metal material 108 positioned differently along the bottom portion 104 of the rod in order to provide metal-on-metal contact with a flat surface.

The locking mechanism 140 may also include a locking element 144 that is configured to engage the body 142 and the rod 100 so as to force the rod 100 toward the fixation device 150. Like the interface between the rod 100 and the rod seat 146, the interface between the rod 100 and the locking element 144 may be metal-on-metal. For example, the top portion 102 of the rod 100 may be formed by the top portion 120 of the metal infrastructure 108.

Turning next to Figure 4, an I-beam shaped metal infrastructure 408 is illustrated. The metal infrastructure 408 includes a top portion 420, bottom portion 422 and sides 414a and 414b, and at least one lateral channel extending 416a-d therethrough. At least one of the lateral channels 416a-e may be positioned such that it does not extend to the top portion 420 or bottom portion 422 of the metal infrastructure 408. In other words, the lateral channels 416a-e may entirely reside within the metal infrastructure 408. As shown, all of the lateral channels 416a-e are positioned such that they do not extend to the top portion 420 or bottom portion 422 of the metal infrastructure 408. In addition, the lateral channels 416a-e may have varying dimensions. For example, the lateral channel 416d may be larger than the lateral channel 416a. Varying the size and location of the lateral channels 416a-e may change the bending properties of the metal infrastructure 408, and thus, the rod. The metal infrastructure 408 is preferably similar in dimension and shape to the metal infrastructure 108. As such, the top portion 420 of the metal infrastructure 408 may have a generally concave shape and the bottom portion 422 of the metal infrastructure 408 may have a generally convex shape.

Turning next to Figure 5, a star-shaped metal infrastructure 508 is illustrated. The metal infrastructure 508 is similar in design and shape to the metal infrastructures 108 and 408 and includes a top portion 520, bottom portion 522 and sides 514a and 514b, and at least one lateral channel extending 516a-e therethrough.

Turning next to Figure 6, a generally cylindrical metal infrastructure 608 is illustrated. The metal infrastructure 608 is similar in design and shape to the metal infrastructures 108, 408 and 508 and includes a top portion 620, bottom portion 622 and sides 614a and 614b, and at least one lateral channel extending 616a-e therethrough. In addition, the metal infrastructure 608 includes an internal axial channel 618, which may intersect with one or more of the lateral channels 616a-e.

At least one of the lateral channels 416a-e may be positioned such that it does not extend to the top portion 420 or bottom portion 422 of the metal infrastructure 408. In other words, the lateral channels 416a-d may entirely reside within the metal infrastructure 408. As shown, all of the lateral channels 416a-e are positioned such that they do not extend to the top portion 420 or bottom portion 422 of the metal infrastructure 408. In addition, the lateral channels 416a-e may have varying dimensions. For example, the lateral channel 116d may be larger than the lateral channel 416a. Varying the size and location of the lateral channels 416a-e may change the bending properties of the metal infrastructure 408, and thus, the rod. The metal infrastructure 408 is preferably similar in dimension and shape to the metal infrastructure 108. As such, the top portion 420 of the metal infrastructure 408 may have a generally concave shape and the bottom portion 422 of the metal infrastructure 408 may have a generally convex shape.

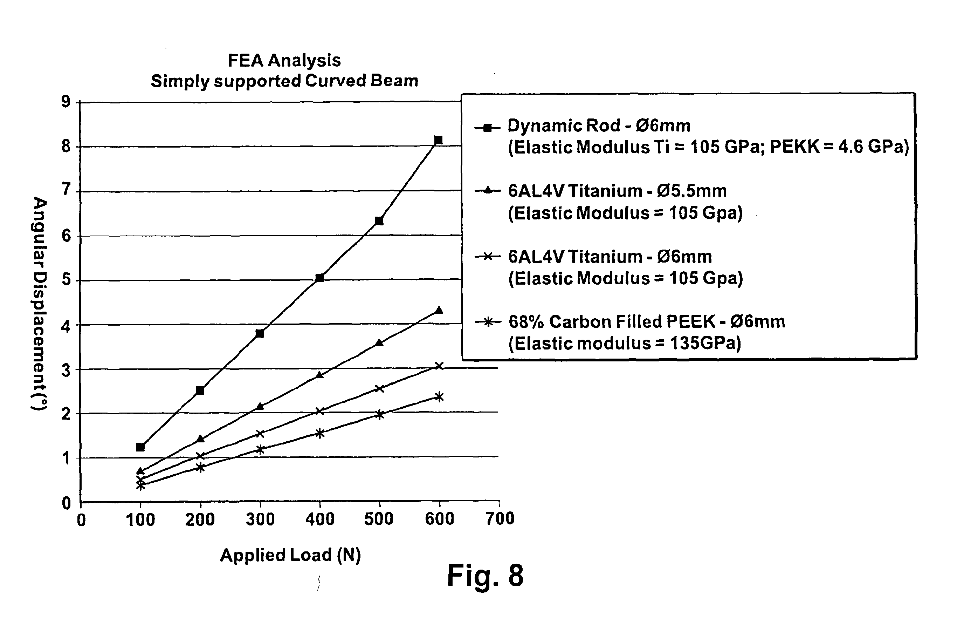

Turning next to Figures 7 and 8, the bending properties of an exemplary rod according to the present invention are illustrated. Figure 7 illustrates a testing procedure for determining bending properties of a rod. Rod 100 is shown to illustrate the testing procedure. As shown, the rod 100 is placed upside down on a central support 702 such that top portion 102 of the rod 100 rests on the support 702. The rod 100 has a known length, which is approximately 10.2cm, (4 inches), and a known diameter, which is 6 mm, for the analysis illustrated in Figure 8. A downward force F is then applied to bottom portion 104 of the rod 100 near the sides 106a and 106b and the angular displacement of the rod 100 is measured.

Preferably, the rod 100 is less resistant to bending when is applied as illustrated in Figure 7 than it is when the rod 100 is flipped over and a downward force is applied to top portion 102 of the rod 100 near the sides 106a and 106b. In other words, the same force F causes less angular displacement when applied to the top portion 102 than when applied to the bottom portion 104. In addition, the rod 100 may have a different bending moment from side to side than from top to bottom. The rod 100 may also have a different bending moment from side to side than from bottom to top.

As can be seen in Figure 8, the rod 100 shows more flexibility than the titanium and carbon filled PEEK rods tested. Indeed, the rod 100 preferably exhibits bending properties, namely angular displacement resulting from an applied load, that are similar to that of a 6 mm rod formed from material having an elastic modulus of greater than or equal to about 10 GPa and less than or equal to about 70 GPa. For example, the rod 100 may exhibit bending properties that are similar to that of a 6 mm rod formed from material having an elastic modulus of greater than or equal to about 15 GPa and less than or equal to about 40 GPa. In contrast, a solid 6 mm and a solid 5.5 mm Ti/6Al/4V rod having the same general shape as the rod 100 are much less flexible due to stiffness of Ti/6Al/4V, which has an elastic modulus of approximately 105 GPa.

While the present invention has been described in association with exemplary embodiments, the described embodiments are to be considered in all respects as illustrative and not restrictive. Such other features, aspects, variations, modifications, and substitution of equivalents may be made without departing from the scope of this invention which is intended to be limited only by the scope of the following claims. Also, it will be appreciated that features and parts illustrated in one embodiment may be used, or may be applicable, in the same or in a similar way in other embodiments.

Although the invention has been shown and described with respect to certain embodiments, it is obvious that certain equivalents and modifications may be apparent to those skilled in the art upon the reading and understanding of the specification. The present invention includes all such equivalents and modifications, and is limited only by the scope of the following claims.

## Claims

1. A rod (100) for use with spinal fixation assemblies comprising:
a metal infrastructure (108, 408, 508, 608) comprising:
a top portion (120, 420, 520, 620),
a bottom portion (122, 422, 522, 622), and
an elastomeric material (110) partially encapsulating the metal infrastructure (108, 408, 508, 608) such that at least part of the top portion (120, 420, 520, 620) of the metal infrastructure (108, 408, 508, 608) or at least part of the bottom portion (122, 422, 522, 622) of the metal infrastructure (108, 408, 508, 608) forms part of the surface of the rod (100) to permit a metal to metal contact surface between the rod (100) and the spinal fixation assembly, **characterized in that** the metal intrastracture further comprises [at least one lateral channel extending (116a-d, 416a-e, 516a-e, 616a-e) through the metal inßrastructure and entirely residing within the metal infrastructure; and **in that** the clastomeris material substantially filling the at least one lateral channel (116a-d, 416a-e, 516a-e, 616a-e) extending through the metal infrastructure (108, 408, 508, 608).

2. A spinal fixation assembly comprising:
a rod (100) according to claim 1; and
a locking mechanism (140a-b) configured to receive the rod (100) and engage part of the surface of the bottom portion (104) of the metal intrastructure.

3. The rod (100) of any one of the preceding claims wherein at least part of the rod (100) exhibits bending properties similar to a 6 mm rod formed from material having an elastic modulus of greater than or equal to about 10 GPa and less than or equal to about 70 GPa.

4. The rod (100) of any one of the preceding claims wherein the rod (100) has a different bending moment from side to side than bottom to top.

5. The rod (100) of any one of the preceding claims wherein at least part of the rod (100) is more resistant to bending in a first direction than in second direction opposite the first direction.

6. The rod (100) of any one of the preceding claims wherein the metal infrastructure (108, 408, 508, 608) does not resemble a cylinder.

7. The rod (100) of claim 6 wherein a cross section of at least part of the metal infrastructure (108, 408, 508, 608) is generally I-beam shaped, generally star-shaped, or generally U-shaped.

8. The rod (100) of claim 7 wherein the bottom portion (122) of the metal infrastructure (108; 408, 508, 608) comprises an axial channel (118, 618).

9. The rod (100) of any one of the preceding claims wherein the center of gravity (136) along at least a portion of the length of the metal infrastructure (108) is not located at the geometric center (134) of the portion of the length of the metal infrastructure (108).

10. The rod (100) of one of the preceding claims wherein the rod (100) is slightly curved such that part of the top portion (102) has a generally concave shape and part of the bottom portion (104) has a generally convex shape.

## Patentansprüche

1. Stange (100) für eine Verwendung mit spinalen Fixierungsanordnungen, die aufweist:
eine Metallinfrastruktur (108, 408, 508, 608), die aufweist:
einen oberen Abschnitt (120, 420, 520, 620);
einen unteren Abschnitt (122, 422, 522, 622); und
ein elastomeres Material (110), das teilweise die Metallinfrastruktur (108, 408, 508, 608) so einkapselt, dass mindestens ein Teil des oberen Abschnittes (120, 420, 520, 620) der Metallinfrastruktur (108, 408, 508, 608) oder mindestens ein Teil des unteren Abschnittes (122, 422, 522, 622) der Metallinfrastruktur (108, 408, 508, 608) einen Teil der Oberfläche der Stange (100) bildet, um eine Metall-Metall-Kontaktfläche zwischen der Stange (100) und der spinalen Fixierungsanordnung zu gestatten, **dadurch gekennzeichnet, dass**
die Metallinfrastruktur außerdem mindestens einen seitlichen Kanal (116a-d, 416a-e, 516a-e, 616a-e) aufweist, der sich durch die Metallinfrastruktur erstreckt und sich vollständig innerhalb der Metallinfrastruktur befindet, und dadurch, dass das elastomere Material im Wesentlichen den mindestens einen seitlichen Kanal (116a-d, 416a-e, 516a-e, 616a-e) füllt, der sich durch die Metallinfrastruktur (108, 408, 508, 608) erstreckt.

2. Spinale Fixierungsanordnung, die aufweist:
eine Stange (100) nach Anspruch 1; und
einen Feststellmechanismus (140a-b), der ausgebildet ist, um die Stange (100) aufzunehmen, und um mit einem Teil der Oberfläche des unteren Abschnittes (104) der Metallinfrastruktur in Eingriff zu kommen.

3. Stange (100) nach einem der vorhergehenden Ansprüche, bei der mindestens ein Teil der Stange (100) Biegeeigenschaften gleich einer Stange von 6 mm zeigt, die aus Material mit einem Elastizitätsmodul von mehr als oder gleich etwa 10 GPa und kleiner als oder gleich etwa 70 GPa gebildet wird.

4. Stange (100) nach einem der vorhergehenden Ansprüche, bei der die Stange (100) ein abweichendes Biegemoment von Seite zu Seite als von unten nach oben aufweist.

5. Stange (100) nach einem der vorhergehenden Ansprüche, bei der mindestens ein Teil der Stange (100) in einer ersten Richtung biegebeständiger ist als in einer zweiten Richtung entgegengesetzt der ersten Richtung.

6. Stange (100) nach einem der vorhergehenden Ansprüche, bei der die Metallinfrastruktur (108, 408, 508, 608) nicht einem Zylinder ähnelt.

7. Stange (100) nach Anspruch 6, bei der ein Querschnitt von mindestens einem Teil der Metallinfrastruktur (108, 408, 508, 608) im Allgemeinen doppel-I-trägerförmig, im Allgemeinen sternförmig oder im Allgemeinen U-förmig ist.

8. Stange (100) nach Anspruch 7, bei der der untere Abschnitt (122) der Metallinfrastruktur (108, 408, 508, 608) einen axialen Kanal (118, 618) aufweist.

9. Stange (100) nach einem der vorhergehenden Ansprüche, bei der sich der Schwerpunkt (136) längs mindestens eines Abschnittes der Länge der Metallinfrastruktur (108) nicht im geometrischen Mittelpunkt (134) des Abschnittes der Länge der Metallinfrastruktur (108) befindet.

10. Stange (100) nach einem der vorhergehenden Ansprüche, bei der die Stange (100) etwas gebogen ist, so dass ein Teil des oberen Abschnittes (102) eine im Allgemeinen konkave Form und ein Teil des unteren Abschnittes (104) eine im Allgemeinen konvexe Form aufweist.

## Revendications

1. Tige (100) destinée à être utilisée avec des assemblages de fixation de la colonne vertébrale, comprenant :
une infrastructure métallique (108, 408, 508, 608), comprenant :
une partie supérieure (120, 420, 520, 620) ;
une partie inférieure (122, 422, 522, 622) ; et
un matériau élastomère (110), encapsulant en partie l'infrastructure métallique (108, 408, 508, 608), de sorte qu'au moins une partie de la partie supérieure (120, 420, 520, 620) de l'infrastructure métallique (108, 408, 508, 608) ou au moins une partie de la partie inférieure (122, 422, 522, 622) de l'infrastructure métallique (108, 408, 508, 608) fait partie de la surface de la tige (100), pour établir une surface de contact métal sur métal entre la tige (100) et l'assemblage de fixation de la colonne vertébrale, **caractérisée en ce que**
l'infrastructure métallique comprend en outre au moins un canal latéral (116a-d, 416a-d, 516a-e, 616a-e), s'étendant à travers l'infrastructure métallique et résidant complètement dans l'infrastructure métallique, et **en ce que** le matériau élastomère remplit le au moins un canal latéral (116a-d, 416a-d, 516a-e, 616a-e) s'étendant à travers l'infrastructure métallique (108, 408, 508, 608).

2. Assemblage de fixation de la colonne vertébrale, comprenant :
une tige (100) selon la revendication 1 ; et
un mécanisme de verrouillage (140a-b), configuré de sorte à recevoir la tige (100) et à s'engager dans une partie de la surface de la partie inférieure (104) de l'infrastructure métallique.

3. Tige (100) selon l'une quelconque des revendications précédentes, dans laquelle au moins une partie de la tige (100) présente des propriétés similaires à une tige de 6 mm formée à partir d'un matériau ayant un module d'élasticité supérieur ou égal à environ 10 GPa et inférieur ou égal à environ 70 GPa.

4. Tige (100) selon l'une quelconque des revendications précédentes, dans laquelle la tige (100) présente un moment de flexion différent d'un côté vers l'autre que du bas vers le haut.

5. Tige (100) selon l'une quelconque des revendications précédentes, dans laquelle au moins une partie de la tige (100) est plus résistante à la flexion dans une première direction que dans une deuxième direction, opposée à la première direction.

6. Tige (100) selon l'une quelconque des revendications précédentes, dans laquelle l'infrastructure métallique (108, 408, 508, 608) ne ressemble pas à un cylindre.

7. Tige (100) selon la revendication 6, dans laquelle une section transversale d'au moins une partie de l'infrastructure métallique (108, 408, 508, 608) a en général une forme de poutre en I, en général une forme en étoile, ou en général une forme en U.

8. Tige (100) selon la revendication 7, dans laquelle la partie inférieure (122) de l'infrastructure métallique (108, 408, 508, 608) comprend un canal axial (118, 618).

9. Tige (100) selon l'une quelconque des revendications précédentes, dans laquelle le centre de gravité (136) le long d'au moins une partie de la longueur de l'infrastructure métallique (108) n'est pas situé au niveau du centre géométrique (134) de la partie de la longueur de l'infrastructure métallique (108).

10. Tige (100) selon l'une des revendications précédentes, dans laquelle la tige (100) est légèrement courbée, de sorte qu'une partie de la partie supérieure (102) a une forme généralement concave, une partie de la partie inférieure (104) ayant une forme généralement convexe.
